Europäisches Patentamt

European Patent Office

Office européen des brevets

(11) Veröffentlichungsnummer: **0 469 441 A1**

## EUROPÄISCHE PATENTANMELDUNG

(21) Anmeldenummer: **91112380.0**

(22) Anmeldetag: **24.07.91**

(51) Int. Cl.5: **A61B 17/56**, A61B 17/58, A61F 2/08

(30) Priorität: **30.07.90 DE 4024148**

(43) Veröffentlichungstag der Anmeldung:
**05.02.92 Patentblatt 92/06**

(84) Benannte Vertragsstaaten:
**DE ES FR GB IT**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Illgner, Andreas, Dr. med.**
**Ressmeyer Hof 8**
**W-3000 Hannover 51(DE)**
Erfinder: **Zwipp, Hans, Prof, Dr. med.**
**Birkenweg 1e**
**W-3000 Hannover 51(DE)**

(54) **Schrauben für Gelenkoperationen.**

(57) Schrauben aus keramischem Material sind zur Fixierung von Transplantaten bei der chirurgischen Rekonstitution von Gelenkbändern geeignet.

Abbildung 1

Gegenstand der Erfindung sind Schrauben zur Fixierung von Gelenkbändern.

Als Folge von Unfällen, z.B. von Sport- und Verkehrsunfällen, kommt es häufig zu Rissen von Gelenkbändern, insbesondere von Kreuzbändern des Kniegelenkes. Sind diese Abrisse nahe der Insertionsstelle am Knochen, so werden bei der chirurgischen Rekonstitution üblicherweise Transplantate benutzt, die mit Schrauben fixiert werden. Derartige Schrauben (Stanzschrauben, englisch: interference screws) werden entsprechend dem Stand der Technik aus Metallen gefertigt (Arthroscopy: The Journal of Arthroscopic and Related Surgery 5 (3) (1989), 225-226). Da diese Schrauben in der Regel im Patienten verbleiben, werden hohe Anforderungen an Bioverträglichkeit, Festigkeit und Langzeitstabilität gestellt. In Bezug auf Verträglichkeit (Allergien) und Korrosionsfestigkeit ist dies nicht immer gewährleistet.

Der Erfindung lag daher die Aufgabe zugrunde, Schrauben mit gegenüber dem Stand der Technik verbesserten Eigenschaften für die Fixierung von Transplantaten bei der chirurgischen Rekonstitution von Gelenkbändern zur Verfügung zu stellen.

Überraschenderweise wurde nun gefunden, daß für den genannten Zweck Schrauben aus keramischem Material sehr gut eingesetzt werden können, da sie neben einer sehr guten Bioverträglichkeit und Langzeitstabilität auch eine hohe Festigkeit besitzen. Als ein weiterer Vorteil ergibt sich, daß keramische Schrauben transparent für Röntgenstrahlen sind, und sich deswegen bei späteren Röntgenaufnahmen des Patienten nur mit geringem Kontrast abbilden. Viele Patienten reagieren betroffen, wenn sie bei derartigen Gelegenheiten die Röntgenbilder mit den massiven (Länge ca. 3 cm; Durchmesser ca. 0,6 cm) Metallteilen sehen. Die erfindungsgemäßen Schrauben hingegen sind im Röntgenbild kaum sichtbar.

Gegenstand der Erfindung sind daher Schrauben für die Fixierung von Gelenkbändern, dadurch gekennzeichnet, daß sie aus keramischem Material bestehen.

Gegenstand der Erfindung ist auch die Verwendung von keramischem Material zur Herstellung von Schrauben zur chirurgischen Rekonstitution von zerstörten Gelenkbändern.

Aus dem Stand der Technik sind zwar schraubenförmige keramische Implantate für andere Zwecke bekannt (DE 25 49 114, DE 25 40 077, JP Offenlegung 62-268553 oder J. of Biomedical Materials Research 14 (1980), 597-605). Diese Ausführungsformen werden jedoch für andere Zwecke eingesetzt und erfüllen nicht die Voraussetzungen für die Verwendung zur Fixierung von Transplantaten bei der chirurgischen Rekonstitution von Gelenkbändern, wie sie erfindungsgemäß beansprucht wird. Für die Verwendung als Stanzschraube sind einige Formelemente essentiell; dazu gehören die Formgebung ohne Schraubenkopf, die Schraubenspitze und ein Gewinde, das sich über die gesamte Länge des Schraubenkörpers erstreckt.

Die erfindungsgemäße Schraube ist deswegen nach Art einer Stanzschraube geformt und besteht aus einem zylindrischen Grundkörper, der sich an einem Ende verjüngt und der am anderen Ende so gestaltet ist, daß die Schraube mit einem geeigneten Werkzeug gedreht werden kann. Für diesen Zweck sind verschiedene Ausführungsformen in der Technik bekannt und können erfindungsgemäß verwendet werden; zu diesen gehören Schlitze, Kreuzschlitze, Innen- und Außensechskantformen (auch entsprechende Vier- und Dreikantformen). Wichtig ist, daß diese Teile nicht seitlich über den übrigen Schraubenkörper herausragen. Das Gewinde der Schraube erstreckt sich über ihre gesamte Länge. Insgesamt wird eine keramikgerechte Formgebung, z.B. unter Vermeidung von scharfen Kanten und spitzen Kerben, vorgezogen. Die Abmessungen der Schraube richten sich danach, für welches Gelenk sie vorgesehen ist. Für die Verwendung am Kniegelenk sind Schrauben mit einer Länge von 2 bis 4 Zentimetern und einem Durchmesser von 0,5 bis 0,9 Zentimetern üblich.

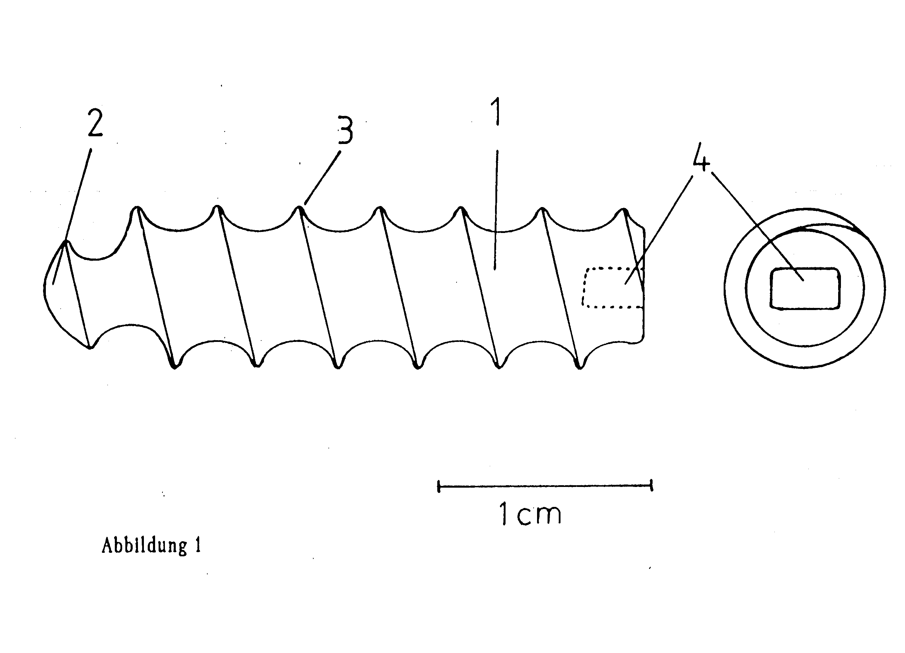

Eine typische Ausführungsform der Erfindung ist in Abbildung 1 dargestellt. Darin ist mit 1 der zylindrische und mit 2 die Schraubenspitze bezeichnet. Der Gewindegang, der sich über die gesamte Länge des Schraubenkörpers erstreckt, ist mit 3 bezeichnet. Mit 4 ist eine Aufnahme für ein Werkzeug bezeichnet, beispielhaft ein Schlitz.

Erfindungsgemäß besteht die Schraube aus einem keramischen Material. Als solches ist ein metalloxidhaltiges oder auch nichtoxidisches Material zu verstehen, das bei Temperaturen zwischen 600 und 2000 Grad Celsius gesintert wird. Das keramische Material besteht beispielsweise aus Aluminiumoxid, Zirkoniumoxid, Titandioxid, Siliciumcarbid oder Siliciumnitrid, vorzugsweise aus Aluminiumoxid.

Zur Herstellung der Schraube dienen in der Technik bekannte und dem Fachmann geläufige Verfahren. Diese sind in der Literatur beschrieben: Technische Keramische Werkstoffe; Herausgeber J. Kriegesmann, Deutscher Wirtschaftsdienst (1989) und Krause, E. et al.: Technologie der Keramik, VEB Verlag für Bauwesen, Berlin (1988).

Wegen der Verwendung als Implantat wird die Schraube vorzugsweise im Anschluß an die Herstellung nach an sich bekannten Verfahren sterilisiert, z.B. durch Autoklavieren bei ca. 120 °C mit überhitztem Wasserdampf für 30 bis 200 Minuten oder durch Erhitzen auf 160 bis 200 °C für 60 bis 300 Minuten, und dann steril verpackt.

Nach dem beschriebenen Verfahren hergestellte Schrauben werden bei chirurgischen Eingriffen zur Rekonstitution von Gelenkbändern benutzt. Sind Gelenkbänder nahe ihrer Insertionsstelle am Knochen abgerissen, so wird zunächst ein Transplantat (typischerweise als Autotransplantat) bestehend aus einem knöchernen und einem faserigen Anteil chirurgisch gewonnen. Dieses Transplantat wird dann mit seinem knöcherigen Teil in einer Bohrung an der ursprünglichen Insertionsstelle mittels einer Schraube (der sogenannten Stanzschraube) befestigt. Das abgerissene Gelenkband wird mit dem faserigen Anteil des Transplantates vernäht. Derartige Operationen sind bei vielen Gelenken möglich; bedeutend ist z.B. die Rekonstitution eines Bandes des Sprunggelenkes oder eines Bandes des Kniegelenkes (z.B. eines Kreuzbandes).

## Beispiele

In den nachfolgenden Beispielen bedeuten "übliche Form" eine Form entsprechend der Abbildung und eine Länge von 3 cm, einen Außendurchmesser von 0,6 cm und einen Kerndurchmesser von 0,4 cm und "übliche Nachbehandlung" das Sterilisieren der Schraube mittels Dampf bei ca. 120 °C, bei ca. 2 bar und für ca. 60 Minuten und anschließender steriler Verpackung. Diese Schraube wird bestimmungsgemäß bei der chirurgischen Rekonstitution eines Kreuzbandes des Kniegelenkes benutzt.

## Beispiel 1

Aluminiumoxidpulver wird mit Polyethylengranulat und Epoxidharz gemischt. Die Rezeptur beträgt dabei 65 Vol.-% Oxidpulver, 25 Vol.-% Polyethylen, 10 Vol.-% Epoxidharz. Die Masse wird in einer Spritzgußmaschine auf 150 °C erhitzt und unter einem Druck von 1,5 kbar in eine wassergekühlte Metallform gespritzt. Bei der Dimensionierung des Spritzgußwerkzeugs wird die Schwindung, die beim späteren Sintern auftritt, berücksichtigt.

Durch das Formwerkzeug entstandene Grate und der Anguß werden mechanisch entfernt. Der Kunststoffanteil wird bei 350 °C ausgebrannt und die Schraube bei 1800 °C gesintert.

## Beispiel 2

Titandioxidpulver wird mit Polyethylengranulat und Epoxidharz gemischt. Die Rezeptur beträgt dabei 65 Vol.-% Oxidpulver, 25 Vol.-% Polyethylen, 10 Vol.-% Epoxidharz. Die Masse wird in einer Spritzgußmaschine auf 150 °C erhitzt und unter einem Druck von 1,5 kbar in eine wassergekühlte Metallform gespritzt. Bei der Dimensionierung des Spritzgußwerkzeugs wird die Schwindung, die beim späteren Sintern auftritt, berücktsichtigt.

Durch das Formwerkzeug entstandene Grate und der Anguß werden mechanisch entfernt. Der Kunststoffanteil wird bei 350 °C ausgebrannt und die Schraube bei 1700 °C gesintert.

## Beispiel 3

Aus einem käuflich erhältlichen massiven Aluminiumoxidstab wird die Schraubenform und das Gewinde mit Diamantwerkzeugen mit feiner Körnung auf einer Drehmaschine gedreht. In einem zweiten Arbeitsgang wird der Schlitz zur Aufnahme des Werkzeugs eingefräst.

## Patentansprüche

1. Schrauben für die Fixierung von Gelenkbändern, dadurch gekennzeichnet, daß sie aus keramischem Material bestehen.

2. Verwendung von keramischem Material zur Herstellung von Schrauben zur chirurgischen Rekonstitution von zerstörten Gelenkbändern.

3. Verwendung von Schrauben aus keramischem Material bei der chirurgischen Rekonstitution von zerstörten Gelenkbändern.

Abbildung 1

1 cm

EP 0 469 441 A1

Europäisches
Patentamt

**EUROPÄISCHER TEILRECHERCHENBERICHT** Nummer der Anmeldung

der nach Regel 45 des Europäischen Patentübereinkommens für das weitere Verfahren als
europäischer Recherchenbericht gilt

EP 91 11 2380

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| Y | WO-A-8 901 767 (WARREN) <br> * Seite 6, Zeilen 26-29; Seite 11, Zeilen 23-26; Figur 1 * <br> --- | 1,2 | A 61 B 17/56 <br> A 61 B 17/58 <br> A 61 F 2/08 |
| Y,D | JOURNAL OF BIOMEDICAL MATERIALS RESEARCH, Band 14, 1980, Seiten 597-605, John Wiley & Sons, Inc.; H. KAWAHARA et al.: "Single crystal alumina for dental implants and bone screws" <br> * Seite 599, Zeilen 9-11; Figur 4 * <br> --- | 1,2 | |
| A | US-A-4 927 421 (GOBLE et al.) <br> * Spalte 5, Zeilen 7-13; Figur 1 * <br> --- | 1 | |
| A | EP-A-0 373 733 (IMZ) <br> * Spalte 3, Zeile 46 – Spalte 4, Zeile 29; Figur 2 * <br> ----- | 1 | |

| | RECHERCHIERTE SACHGEBIETE (Int. Cl.5) |
|---|---|
| | A 61 B <br> A 61 F |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1,2
Unvollständig recherchierte Patentansprüche:
Nicht recherchierte Patentansprüche: 3
Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| DEN HAAG | 04-11-1991 | MOERS R.J. |

EPO FORM 1503 03.82 (P0409)